# EUROPÄISCHE PATENTSCHRIFT

(11) **EP 3 302 340 B1**
(45) Veröffentlichungstag und Bekanntmachung des Hinweises auf die Patenterteilung: **03.04.2019**
(21) Anmeldenummer: 16724634.7
(22) Anmeldetag: 23.05.2016
(51) Int. Cl.: A61B 90/90, A61B 90/98, A61B 50/30, G06K 7/10

(54) **CHIRURGISCHES BEHÄLTERINHALT-ERFASSUNGSSYSTEM**
SURGICAL SYSTEM FOR DETECTING THE CONTENT OF A CONTAINER
SYSTÈME DE DÉTECTION DE CONTENU DE CONTENANT CHIRURGICAL

(30) Priorität: 26.05.2015 DE 102015108264
(43) Veröffentlichungstag der Anmeldung: 11.04.2018
(73) Patentinhaber: Aesculap AG, 78532 Tuttlingen (DE)
(72) Erfinder: WEHRLE, Christian, 78269 Volkertshausen (DE); NONNENMANN, Martin, 78573 Wurmlingen (DE)
(74) Vertreter: Winter, Brandl, Fürniss, Hübner, Röss, Kaiser, Polte - Partnerschaft mbB
(86) Internationale Anmeldenummer: PCT/EP2016/061589
(87) Internationale Veröffentlichungsnummer: WO 2016/188959

(56) Entgegenhaltungen:
- WO-A1-2009/003231
- DE-A1-102006 060 176
- DE-A1-102007 056 261
- DE-A1-102011 050 333
- US-A1- 2006 043 179
- US-A1- 2006 145 871
- US-A1- 2006 244 593
- US-A1- 2007 160 494
- US-A1- 2008 150 722
- US-A1- 2014 125 482

## Beschreibung

Die Erfindung betrifft ein chirurgisches oder medizinisches Behälterinhalt-Erfassungssystem.

Erfassungssysteme und Erfassungsverfahren für chirurgische oder medizinische Instrumente und Materialien sind beispielsweise aus der DE 100 14 542A1bekannt. Sie können insbesondere zur Überwachung und Steuerung eines Materialflusses in einem Krankenhaus eingesetzt werden. Ein Verfahren und eine Einrichtung zur Überwachung und Steuerung des Materialflusses in einem Krankenhaus sind aus der DE 196 14 719 A1 bekannt.

Aus der DE 10 2011 050 333 A1 ist ferner ein Behälterinhalt-Erfassungssystem mit einer Behälterinhaltsensoreinrichtung zum Anordnen in oder an einem Sterilisationsbehälter bekannt. Die Behälterinhaltsensoreinrichtung umfasst einen Träger und mindestens einen am Träger angeordneten oder ausgebildeten Sensor zum Detektieren eines Identifizierungselements, welches an einem im Sterilisationsbehälter gelagerten Gegenstand zu dessen Identifikation angeordnet oder ausgebildet ist. Der Behälterinhalt wird sodann durch ein spezielles externes Lesegerät erfasst. Des Weiteren sei noch auf die US2006/244593 A1, auf welcher der Oberbegriff von Anspruch 1 beruht, US2007/160494A1, WO 2009/003231 A1 und US2008/150722A1 hingewiesen, aus denen jeweils ein Instrumenten-Erfassungssystem bekannt ist.

Nachteilig bei einer bekannten Anordnung ist jedoch, dass das externe Lesegerät nicht an jedem Ort verfügbar und daher lediglich eine Momentaufnahme zum Zeitpunkt einer jeweils letzten Erfassung bereitstellbar ist. Jederzeit aktuelle Daten über beispielsweise einen letzten Sterilisationsprozess, einen aktuellen Inhalt und dergleichen, sind nicht verfügbar. Die Verfügbarkeit derartiger Informationen und/oder Daten ist jedoch wichtig, da jederzeit feststellbar sein muss, welche Gegenstände sich in einem Sterilisationsbehälter befinden. Es muss vermieden werden, mangels Lesegerät einen Sterilisationsbehälter zur Überprüfung öffnen zu müssen, insbesondere wenn der Sterilisationsbehälter mit seinem Inhalt einen Sterilisationsprozess durchlaufen hat, da andernfalls der Sterilisationsbehälter nach dem Öffnen nicht mehr steril wäre.

Der Erfindung liegt daher als eine Aufgabe zugrunde, eine Vorrichtung/Anlage/System bereitzustellen, mit welcher der Inhalt eines Sterilisationsbehälters jederzeit einfach und sicher bestimmbar ist, und die eine Nachverfolgung von Sterilisationsbehältern in ihrer Verwendungsumgebung erlaubt.

Erfindungsgemäß wird diese Aufgabe durch ein chirurgisches bzw. medizinisches Behälterinhalt-Erfassungssystem/Anlage mit den in Anspruch 1 angegebenen Merkmalen gelöst. Vorteilhafte Weiterbildungen der Erfindung sind Gegenstand der beigefügten Unteransprüche.

Gemäß einer grundlegenden Idee der Erfindung wird dazu ein Lesegerät, beispielsweise ein RFID-Lesegerät, in oder an einem Sterilisationsbehältersystem/-anlage so verbaut, so dass dieses die darin abgelegten und daraus herausgenommenen Instrumente erfassen kann. Das Lesegerät ist mit einer Energieversorgungseinrichtung sowie einem drahtlos arbeitenden (Wireless-) Funkmodul verbunden. Mithilfe des Funkmoduls können erfasste RFID-Daten an ein Endgerät (z.B. ein Tablet-Computer, ein Notebook-Computer, ein Smartphone oder dergleichen) übermittelt und dort ausgewertet werden. Das Lesegerät, das Funkmodul sowie eine Energieversorgungseinrichtung zu deren Versorgung sind derart zu einer Einheit vergossen oder zusammengefasst, dass diese Einheit mit sterilisiert werden kann. Vorzugsweise ist diese Einheit mattenförmig ausgebildet. Das Funkmodul weist eine Sendeleistung auf, die so bemessen ist, dass eine Datenübertragung aus dem Inneren des Sterilisationsbehälters nach außen erfolgen kann. Insbesondere kann das Funkmodul mittels Niedrigenergie-Bluetooth mit geringer Stromaufnahme arbeiten. Ferner kann das Funkmodul eine Relaisfunktion zum Einschalten und/oder Ausschalten des Lesegeräts aufweisen. Ein Temperatur-, Feuchtigkeit- und/oder Druckhöhensensor kann bereitgestellt und ankoppelbar sein. Das Funkmodul kann ferner einen separaten bzw. eigenen Speicher aufweisen, der beispielsweise als EEPROM ausgebildet sein und einen Datenspeicher für beispielsweise Seriennummern, Namen, Sterilisationshistorie, Wartungshistorie und dergleichen bereitstellen kann. Darüber hinaus kann insbesondere zur Nachverfolgung bzw. für ein Tracking von Sterilisationsbehältern eine Niedrigenergie-Bluetooth-Einrichtung (BLE bzw. BT LE; Bluetooth Low Energy) mit einem Antennenarray zur Ortung des Sterilisationsbehälters innerhalb beispielsweise einer Lagereinrichtung vorgesehen sein. Die Energieversorgung kann vorzugsweise über eine Batterie, einen Akkumulator, einen Kondensator (SuperCap) erfolgen, die beispielsweise über eine steckbare Kabelverbindung, induktiv und/oder mittels Thermogeneration aufladbar sein können. Ferner kann eine Richtungserkennung vorgesehen sein, die beispielsweise eine Transportrichtung eines Sterilisationsbehälters erkennbar und zu Nachverfolgungs- und/oder Ortungszwecken heranziehbar macht.

Im Einzelnen wird die Aufgabe gelöst durch ein chirurgisches Behälterinhalt-Erfassungssystem, beinhaltend eine Behälterinhalt-Sensoreinrichtung zum Anordnen in einem Sterilisationsbehälter, welche Behälterinhaltsensoreinrichtung einen Träger und mindestens einen am Träger angeordneten oder ausgebildeten Sensor zum Erfassen mindestens eines Identifizierungselements, welches an einem im Sterilisationsbehälter gelagerten Gegenstand zu dessen Identifikation angeordnet oder ausgebildet ist, umfasst. Ein an dem Träger angeordnetes Trägermodul, das eine Erfassungseinrichtung zur Erfassung mindestens eines erfassten Identifizierungselements aufweist und dazu angeordnet ist, Information über den durch das erfasste Identifizierungselement identifizierten Gegenstand drahtlos nach außerhalb des Sterilisationsbehälters zu übermitteln. Vorteilhaft wird dadurch ein RFID-Lesegerät in/an einem Sterilbehältersystem zur Erfassung abgelegter und/oder herausgenommener, einschließlich sich in einem Behälter befindender, Instrumente bereitgestellt, das eine separate Einheit bildet, die ohne funktionsbezogen mechanische Verbindung zu dem Sterilbehältersystem betreibbar ist und damit optional zu dem Sterilbehältersystem verwendet werden kann.

Bevorzugt beinhaltet die Behälterinhalt-Sensoreinrichtung mindestens eine Datenübermittlungseinrichtung und eine drahtlose Schnittstelle zur Übertragung und/oder Auskopplung von mit dem mindestens einen Sensor erzeugten Erfassungssignalen an eine behälterexterne Auslese- und/oder Auswerteeinheit zum Bestimmen der im Sterilisationsbehälter gelagerten Gegenstände in Abhängigkeit von mit dem mindestens einen Sensor erzeugten Erfassungssignalen. Aufgrund der drahtlosen Übermittlung von Information durch die Gehäusewandung hindurch, welche RFID-basiert aufgrund deren Abschirmwirkung nicht möglich wäre, bestehen hohe Freiheitsgrade bei Wahl, Ausgestaltung und Ankopplung kompatibler Endgeräte. Manuelle Eingriffe an einem Sterilisationsbehälter sind, beispielsweise während einer Lagerung oder Nachverfolgung, nicht erforderlich.

Bevorzugt beinhaltet das Trägermodul zumindest einen Sensor, der eine Transponder-Leseeinrichtung bildet, eine Energieversorgungseinrichtung zur Energieversorgung der Behälterinhalt-Sensoreinrichtung, und/oder eine drahtlos arbeitende Datenübermittlungseinrichtung zur Übermittlung von erfassten Daten und/oder Erfassungssignalen an eine behälterexterne Auslese- und/oder Auswerteeinrichtung, und ist das Trägermodul zu einer dampfsterilisierbaren Einheit vergossen ist. Dadurch, dass die Einheit aus RFID-Lesegerät, Wireless-Modul und Energieversorgungseinheit komplett vergossen ist, kann die gesamte Einheit mit sterilisiert werden. Außerdem kann die gesamte Einheit vorgefertigt und als solche zur Einbettung an einem vorbestimmten Ort am Träger bereitgestellt werden, wodurch sich zusätzliche Freiheitsgrade bei unterschiedlichen Trägergrößen ergeben.

Bevorzugt beinhaltet das Trägermodul eine Temperatur- Feuchtigkeit- und Druckhöhenerfassungseinrichtung. Eine behälterintern verbaute Temperatur- Feuchtigkeit- und Druckhöhene erfassungseinrichtung erleichtert das direkte und präzise Aufzeichnen bzw. Loggen betriebsrelevanter Daten und Parameter, da zumindest während eines laufenden Sterilisationsprozesses der Sterilbehälter in der Regel in einem Sterilisator verriegelt und nicht weiter zugänglich ist.

Bevorzugt beinhaltet das Trägermodul eine Speichereinrichtung zur Speicherung fester Daten und zumindest Zwischenspeicherung erfasster Daten, wobei die Speichereinrichtung dazu angeordnet ist, zumindest Identifikationsinformation des Sterilisationsbehälters, die zumindest eine Seriennummer oder eine Kennung desselben umfassen, fest zu speichern, und/oder zumindest von einer Temperatur- Feuchtigkeit- und Druckhöhenerfassungseinrichtung erzeugte Daten in Bezug auf einen Temperaturverlauf, Feuchtikeitsverlauf und Druckhöhenverlauf an dem Sterilisationsbehälter, die zumindest Information über einen letzten Sterilisationszeitraum oder eine Anzahl von Sterilisationszyklen beinhalten, von dem wenigstens einen Sensor bezüglich der Identifizierungselemente erfasste Daten oder Daten bezüglich einer Sterilisationshistorie und/oder einer Wartungshistorie des Sterilisationsbehälters zwischenzuspeichern. Eine behältereigene Speichereinrichtung bietet Vorteile bei der Aufzeichnung betriebsrelevanter Daten und Parameter und erlaubt eine eindeutige Identifikation eines Sterilisationsbehälters. Bei Vorhandensein mehrerer Sterilisationsbehälter in räumlicher Nähe können durch drahtloses Auslesen lokal gespeicherter Information eine Abfrage über mehrere Behälter hinweg, eine Suche nach einem bestimmten Behälter und dergleichen sicher und einfach durchgeführt werden.

Bevorzugt beinhaltet das Trägermodul eine Relaisfunktion, die für ein Aktivieren und/oder Deaktivieren von zumindest Teilen des Behälterinhalt-Sensorsystems einschließlich des Trägermoduls selbst konfiguriert ist, wobei die Relaisfunktion in Abhängigkeit von an dem Trägermodul erfassten Zustandsänderungen eigenauslösbar oder abhängig oder unabhängig von derartigen Zustandsänderungen fernauslösbar ist. Für eine weitgehend berührungslose Verwendbarkeit ist es vorteilhaft, wenn behälterinterne elektrische Komponenten sich bedarfsweise und zustandsabhängig selbst aktivieren und auch wieder deaktivieren, d.h. zumindest in einen Bereitschaftszustand versetzen können. Beispielsweise kann bei Erfassen eines Temperaturanstiegs selbsttätig ein Datenerfassungs- und Speichervorgang begonnen werden, kann nach Verstreichen einer vorbestimmten Zeitdauer ein Übergang in einen energiesparenden Schlafzustand vorgesehen sein, und kann eine Aufwachfunktion mit nachfolgend vorbestimmter Funktionalität bei drahtloser Ansprache eines Behälters durch ein externes Endgerät und dergleichen implementiert sein. Insgesamt werden Fehlbedienungen reduziert und die Betriebssicherheit des Systems erhöht.

Bevorzugt ist die Energieversorgungseinrichtung für eine Eigenenergieversorgung der Behälterinhalt-Sensoreinrichtung angeordnet und umfasst sie zumindest eine wiederaufladbare Energiespeichereinrichtung, die induktiv und/oder mittels Thermogeneration behälterseitig erzeugter elektrischer Energie speisbar ist. Eine Eigenenergieversorgung erzeugt vorteilhaft die für den Betrieb elektrischer Einrichtungen des Systems erforderliche Energie aus Zustandsänderungen, dem es unterliegt, selbst. Durch die lokale Speicherung werden Relaisfunktionen wie vorstehend erwähnt, Datenhaltefunktionen und dergleichen gewährleistet. Eine Meldeeinrichtung, die dazu ausgelegt ist, bei längerer Nichtverwendung einen in vorbestimmter Weise geringer werdenden Energiespeicherzustand anzuzeigen, beispielsweise eine Meldeleuchte oder ein Summer an einem Sterilisationsbehälter selbst, oder ein automatisches Senden einer Meldeinformation an ein erreichbares Endgerät oder eine zentrale Verwaltungseinrichtung mittels drahtloser Übermittlung zur Benachrichtigung über einen bestimmten Überwachungszustand, kann vorgesehen sein.

Bevorzugt ist das Trägermodul dazu angeordnet, in Verbindung mit einer hochgenauen Hausortung und BLE-Konformität in Bezug auf die Eigenenergieversorgung der Behälterinhalt-Sensoreinrichtung eine Einrichtung zur Sterilisationsbehälter-Nachverfolgung und/oder -Ortung auf der Grundlage eines RSSI-Werts und/oder eine Richtungserkennungseinrichtung bereitzustellen. Intelligentes Design und/oder Softwareroutinen, die in einer auf dem Trägermodul bereitgestellten zentralen Verarbeitungseinrichtung, die beispielsweise eine SOC-Komponente oder ein Prozessor sein kann, ausgeführt werden, können vorteilhaft erforderliche Daten und Informationen liefern und eine Nachverfolgbarkeit, Ortbarkeit und Richtungserkennbarkeit zumindest unterstützen. Insgesamt werden hierdurch Investitionskosten reduziert, denn bereits vorhandene Hardware kann genutzt werden. Durch den Wegfall von Hardwarebeschränkungen sinken zudem Komplexität und Fehleranfälligkeit des RFID Systems, Service und Wartung für eine softwarebasierte Lösung sind zu vernachlässigen. universeller und kostengünstiger.

Bevorzugt ist der mindestens eine Sensor dazu angeordnet ist, Identifizierungselemente in Form von Transpondern zu erfassen. Auf einfache Weise lassen sich beispielsweise Identifizierungselemente in Form von Transpondern detektieren und auswerten, wenn der Detektor in Form einer Transponderleseeinrichtung ausgebildet ist.

Bevorzugt ist der mindestens eine Sensor in Form eines Transponders ausgebildet und in einer Ausnehmung des Trägers aufgenommen. Transponder lassen sich klein, kompakt und kostengünstig zur Ausbildung des Systems bereitstellen. Vorzugsweise ist der Transponder in Form eines RFID-Transponders ausgebildet. Das Akronym RFID basiert auf dem englischen Begriff "Radio-Frequency Identification". Dies lässt sich ins Deutsche übersetzen mit "Identifizierung mit Hilfe elektromagnetischer Wellen". Derartige Transponder sind klein und kostengünstig am Markt verfügbar. Günstigerweise ist ferner der mindestens eine Sensor in einer Ausnehmung des Trägers angeordnet und kann auf diese Weise geschützt werden, beispielsweise vor der Beaufschlagung mit Heißdampf im Verlauf eines Sterilisationsprozesses.

Bevorzugt umgibt der Träger das Trägermodul und den mindestens einen Sensor mindestens teilweise formschlüssig, und ist er aus einem dampfsterilisierbaren Material hergestellt. Ein besonders guter Schutz des mindestens einen Sensors lässt sich erreichen, wenn der Träger den mindestens einen Sensor vollständig umgibt. Eine Stabilität des Trägers lässt sich beispielsweise dadurch verbessern, dass der Träger den mindestens einen Sensor mindestens teilweise formschlüssig umgibt. Vorzugsweise umgibt der Träger den mindestens einen Sensor vollständig formschlüssig. Auf diese Weise lassen sich unerwünschte Hohlräume vermeiden, in welche im ungünstigsten Fall zudem Feuchtigkeit eindringen kann. Um eine Wiederverwendung der Behälterinhaltsensoreinrichtung zu ermöglichen, ist es vorteilhaft, wenn der Träger aus einem dampfsterilisierbaren Material hergestellt ist. Die Behälterinhaltsensoreinrichtung kann dann beispielsweise im Sterilisationsbehälter verbleiben, bevor dieser wieder mit unsterilen Gegenständen bestückt wird, um anschließend einen Sterilisationsprozess zu durchlaufen.

Bevorzugt ist der Träger aus einem Kunststoff hergestellt. Besonders einfach und kostengünstig lässt sich die Behälterinhaltsensoreinrichtung herstellen, wenn der Träger aus einem Kunststoff hergestellt ist. Bei dem Träger kann es sich insbesondere um einen starren oder auch einen flexiblen oder elastischen Träger handeln.

Bevorzugt ist der Träger in Form einer Matte ausgebildet. Günstig ist es, wenn der Träger in Form einer einlegbaren Matte ausgebildet ist. Diese kann insbesondere auch abstehende Noppen aufweisen, um im Sterilisationsbehälter zu lagernde Gegenstände vor Beschädigungen zu schützen. Insbesondere können Noppen stoßdämpfend wirkend ausgebildet sein. Da erfindungsgemäß keine elektrischen oder mechanischen Verbindungen nach außerhalb des Trägers erforderlich sind, eignet sich der mattenförmige Träger als optionaler Systembestandteil, d.h. ein Sterilisationsbehälter kann bedarfsweise mit oder ohne eingelegten Träger verwendet werden. Das Fehlen elektrischer und mechanischer Verbindungen nach außerhalb des Trägers ermöglicht darüber hinaus, einen Träger einer bestimmten Größe bzw. mit bestimmten Abmessungen in einer Mehrzahl unterschiedlich großer Sterilisationsbehälter zu verwenden.

Bevorzugt kann der Träger in Form einer Einlegevorlage ausgebildet sein, auf welcher im Sterilisationsbehälter zu lagernde Gegenstände mindestens schematisch dargestellt sind. Beispielsweise können auf dem Träger Umrisse der Gegenstände abgebildet sein, die einer Bedienperson zeigen beziehungsweise als Anweisung dienen, wo und wie welche Gegenstände im Sterilisationsbehälter zu lagern sind. Die Einlegevorlage kann beispielsweise auch in Form einer Matte ausgebildet sein. Sie muss nicht zwingend starr oder steif ausgebildet sein, sondern sie kann auch flexibel und/oder elastisch ausgebildet sein.

Bevorzugt wird auch, einen Sterilisationsbehälter mit einem Behälterunterteil und einem Behälteroberteil zum Verschließen des Behälterunterteils zu konfigurieren, wobei die Behälterinhalt-Sensoreinrichtung mechanisch verbindungsfrei in den Sterilisationsbehälter einlegbar ist. Eine solche Ausgestaltung ermöglicht beispielsweise in sicherheitskritischem Umfeld auch eine redundante Überprüfung des Inhalts des Sterilisationsbehälters durch Einlegen mehrerer Behälterinhalt-Sensoreinrichtungen, so dass der Ausfall einer der Behälterinhaltsensoreinrichtungen trotzdem noch ermöglicht, den Inhalt des Sterilisationsbehälters zuverlässig zu bestimmen.

Erfindungsgemäß umfasst das System auch einen Siebkorb mit mindestens einer Lagereinrichtung für mindestens einen im Siebkorb zu lagernden Gegenstand, welcher Siebkorb in den Sterilisationsbehälter einbringbar ist. Mit anderen Worten ist der Siebkorb derart ausgebildet, dass er in den Behälterunterteil des Sterilisationsbehälters eingesetzt und der Behälterunterteil anschließend mit dem darin befindlichen Siebkorb mit dem Behälteroberteil verschlossen werden kann.

Erfindungsgemäß ist die Behälterinhalt-Sensoreinrichtung im Siebkorb angeordnet oder gehalten. Mit der Entnahme des Siebkorbs aus beispielsweise dem Behälterunterteil kann somit auch die Behälterinhaltsensoreinrichtung aus dem Sterilisationsbehälter entnommen werden.

Die Erfindung wird nachstehend anhand bevorzugter Ausführungsbeispiele unter Bezugnahme auf die beigefügte Zeichnung näher beschrieben. Die einzige Figur zeigt eine schematische Darstellung eines Behälterinhalt-Erfassungssystems gemäß einem Ausführungsbeispiel der Erfindung.

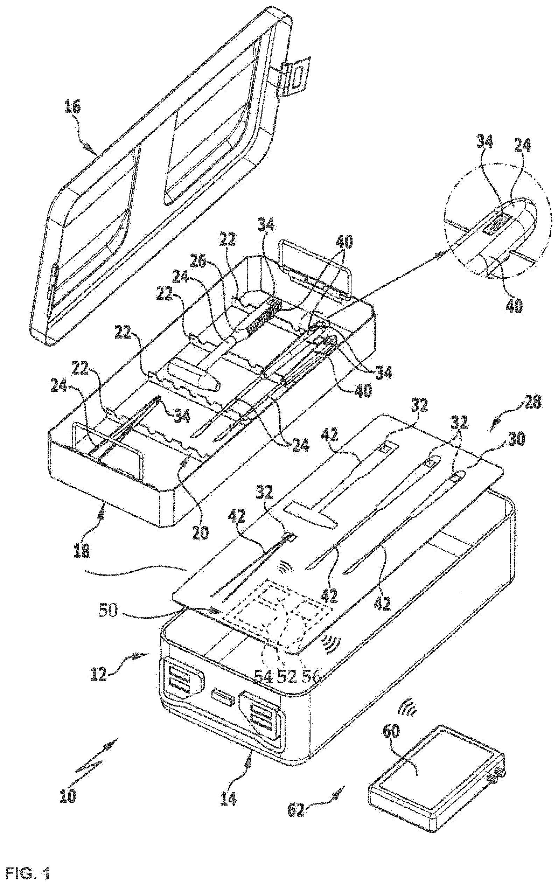

Die Figur 1 zeigt schematisch ein erstes Ausführungsbeispiel eines insgesamt mit dem Bezugszeichen 10 bezeichneten chirurgischen Behälterinhalt-Erfassungssystems bzw. einer chirurgischen Behälterinhalt-Erfassungsanlage. Das Behälterinhalt-Erfassungssystem (Anlage/Vorrichtung) 10 beinhaltet einen Sterilisationsbehälter 12 vorzugsweise mit einem wannenförmigen Behälterunterteil 14 und einem einen Deckel bildenden Behälteroberteil 16 zum Verschließen des Behälterunterteils 14. Der Behälter kann aber auch eine seitliche Beladetür oder dergleichen Zugang haben.

Des Weiteren umfasst/hat das Behälterinhalt-Erfassungssystem 10, nachstehend verkürzt auch nur als System 10 bezeichnet, vorzugsweise einen (separaten) Siebkorb 18 zum Einsetzen in den Behälterunterteil 14. In dem Siebkorb 18 ist vorteilhafter Weise eine Lagereinrichtung 20 vorgesehen, welche mehrere, parallel zueinander angeordnete, streifenförmige Lagerelemente 22 mit Lagerausnehmungen zum definierten Lagern von Gegenständen 24 umfasst/aufweist. Bei den Gegenständen 24 kann es sich insbesondere um in der Figur 1 beispielhaft dargestellte medizinische und chirurgische Instrumente, oder um in der Figur 1 nicht dargestellte Implantate oder Implantatteile handeln.

Des Weiteren umfasst/hat das System 10 eine Behälterinhalt-Sensoreinrichtung 28. Sie umfasst/betrifft einen Träger 30 in Form beispielsweise einer Matte oder Einlegevorlage. An dem Träger 30 sind ein oder mehrere Sensoren 32 zum Detektieren eines Identifizierungselements 34 angeordnet oder ausgebildet. An jedem (einzulegenden) Gegenstand 24 ist wenigstens ein solches Identifizierungselement 34 angebracht, um ihn eindeutig identifizieren zu können. Das Identifizierungselement 34 ist vorzugsweise in Form eines Transponders ausgebildet, welcher beispielsweise in Form eines sogenannten RFID-Chips bereitgestellt sein kann. Vorzugsweise sind die Identifizierungselemente 34 an Griffelementen 40 der Gegenstände angeordnet.

Im Hinblick auf bei RFID erzielbare Reichweiten ist in Bezug auf bei Sterilisationsbehältern/-containern 12 der in Rede stehenden Art auftretenden Größenordnungen und aufgrund der Abschirmwirkung der Behälter- und/oder Siebkorbwandungen vorwiegend zwischen Nahkopplung (close coupling) und Fernkopplung (remote coupling) zu unterscheiden. Bei der Nahkopplung sind Reichweiten von 0 bis 1 cm erzielbar, d.h. die Positionen von Sensor 32 und Identifizierungselement 34 müssen bei einem Lesevorgang genau definiert sein. Durch die in diesem Fall enge Kopplung können dem Identifizierungselement 34 größere Energiemengen bereitgestellt werden, so dass Anwendungen, bei welchen erhöhte Sicherheit maßgeblich ist, vorzugsweise mittels induktiver oder kapazitiver Nahkopplung realisiert werden können. Bei der Fernkopplung betragen erzielbare Reichweiten, in der Regel mittels induktiver Kopplung und passiver Energieversorgung des Identifizierungselements 34 mittels aus dem Magnetfeld einer Leseeinrichtung übertragener Energie, bis zu 1 m.

Demgemäß kann ein einzelner Sensor 52, dessen Funktionsweise grundlegend der des Sensors 32 entsprechen kann, in einem an vorbestimmter Stelle an bzw. in dem Träger 30, beispielsweise in einem Rand- oder Eckbereich desselben, angeordneten, noch zu beschreibenden Trägermodul 50 bereitgestellt sein und mittels beispielsweise Fernkopplung zentral Daten und/oder Information von mehreren Identifizierungselementen 34 an jeweiligen Gegenständen 24 erfassen. Alternativ können bei Anwendung einer Nahkopplung mehrere Sensoren 32 in unmittelbarer Nähe an jeweils den Identifizierungselementen 34 angeordnet und beispielsweise über in dem Träger 30 verlaufende (nicht dargestellte) Leiterverbindungen mit einer (nicht dargestellten) zentralen Verarbeitungseinrichtung in dem Trägermodul 50 verbunden sein. Weiter alternativ ist ferner eine Kombination, d.h. Anordnung sowohl als auch, der vorstehenden Sensoren 32 und 52 darstellbar, mittels welcher beispielsweise fein differenzierte Abstufungen der Behälterinhaltserfassung (beispielsweise erhöhte Sicherheit durch Nahkopplung und jeweils Sensor-Identifizerungselement-Paare nahe an etwa vorrangigen Gegenständen gegenüber Einsparmöglichkeiten durch Fernkopplung unter Entfall von Identifizierungselementen an etwa nachrangigen Gegenständen) erzielbar sind. Gemäß der Erfindung bildet der Siebkorb 18, welcher aus Metall gefertigt ist, selbst eine Antenne. Es wäre aber auch denkbar, dass über separate Antennen das jeweilge RFID-Signal auf den metallischen Korb eingekoppelt wird.

Das Trägermodul 50 umfasst/hat in diesem Ausführungsbeispiel eine Transponder-Leseeinrichtung bzw. RFID-Leseeinrichtung, die durch beispielsweise den Sensor 52 gebildet und dazu ausgelegt sein kann, auch mehrere Identifizierungselemente 34 und/oder Sensoren 32 auszulesen, aber auch Teil einer (nicht gezeigten) zentralen Erfassungs- und Verarbeitungseinheit mit einem Sensorabschnitt und/oder einem Speicherabschnitt bilden kann, eine Energieversorgungseinrichtung 54, und ein drahtlos arbeitendes Funkmodul bzw. Wireless-Modul 56, mittels dem von der TransponderLeseeinrichtung erhaltene (RFID-)Daten an eine Auslese- und/oder Auswerteeinheit 62, beispielsweise ein Endgerät nach Art eines Tablet-Computers, eines Notebook-Computers, eines Smartphones oder dergleichen, außerhalb des Sterilisationsbehälters 12 zur dortigen Auswertung übermittelbar sind.

Das Trägermodul 50 weist darüber hinaus vorzugsweise eine (nicht dargestellte) separate Speichereinrichtung auf, beispielsweise ein EEPROM oder einen Flash-Speicher vorbestimmter Größe, zur behälterinternen Speicherung von Daten wie beispielsweise einer Seriennummer, einer Bezeichnung oder Kennung, und/oder Historiendaten betreffend eine Sterilisationshistorie, eine Wartungshistorie und dergleichen.

Ferner weist das Trägermodul 50 vorzugsweise einen (nicht dargestellten) Temperatur- Feuchtigkeit- und Druckhöhenersensor auf, der in das Trägermodul 50 integriert ausgebildet sein kann, zur Aufzeichnung beispielsweise eines letzten Sterilisationszeitraums, einer Anzahl erfolgter Sterilisationszyklen und dergleichen. Von dem Temperatur- Feuchtigkeit- und Druckhöhenersensor erfasste Daten sind in der Speichereinrichtung des Trägermoduls 50 speicherbar.

Des Weiteren weist das Trägermodul 50 vorzugsweise eine Relaisfunktion auf, die für ein intelligentes Aktivieren und/oder Deaktivieren des Systems 10 konfiguriert ist. Das intelligente Aktivieren und/oder Deaktivieren kann beispielsweise eine eigengesteuerte Zustandsänderung bei Erfassung eines vorbestimmten Zustands oder einer vorbestimmten Zustandsänderung, oder eine über die Auslese- und/oder Auswerteeinheit 62 fernausgelöste Zustandsänderung umfassen. Realisierbare Intelligenz kann beispielsweise eine Aufwachfunktion, eine Bereitschaftsfunktion und dergleichen bei korrespondierender Ansprache oder Adressierung durch die Auslese- und/oder Auswerteeinheit 62, eine selbsttätige Aktivierung bei Erfassen beispielsweise eines Einlegens eines ersten Gegenstands 24 oder bei Schließen des Sterilisationsbehälters 12, eine selbsttätige Deaktivierung bei Verstreichen einer vorbestimmten Zeitdauer ohne Änderung, und dergleichen beinhalten.

Im Übrigen kann das Trägermodul 50 für eine Richtungserkennung konfiguriert sein. Stark bevorzugt erfolgt dabei eine Richtungserkennung hardwareunabhängig durch eine Auswertung der Lesedaten einer Leseeinrichtung mittels Software, so dass auch ohne Vorhandensein spezieller Einrichtungen für die Richtungserkennung, wie beispielsweise entsprechend ausgelegte Leser oder Antennen, die Richtungsbestimmung eines Gegenstands erfolgen kann. Eine Funktion zur Erkennung und Filterung von Falschpositiven kann dabei vorgesehen sein. Das Trägermodul 50 ist in diesem Fall für ein Zusammenwirken mit einer vorzugsweise softwarebasiert arbeitenden Richtungserkennungseinrichtung konfiguriert und zur Bereitstellung von Daten für eine Richtungserkennung ausgestattet.

Außerdem kann das Trägermodul 50 einen (nicht gezeigten) Energiespeicher zur Eigenversorgung des Moduls, des Trägers 30, und/oder des Sterilisationsbehälters 12 bzw. des Systems 10 insgesamt umfassen. Der Energiespeicher, beispielsweise eine wiederaufladbare Batterie bzw. ein Akkumulator und/oder ein Kondensator, vorzugsweise ein SuperCap-Kondensator, kann in diesem Fall über eine steckbare Energiezufuhr, mittels induktiver Einkopplung und/oder, insbesondere durch Ausnutzen der während des Sterilisationsprozesses auftretenden Temperaturen und Temperaturänderungen, d.h. der während des Sterilisationsprozesses auftretenden Abwärme, mittels beispielsweise thermogenerierenden Peltier-Elementen für die Erzeugung elektrischer Energie gespeist werden. Insbesondere Thermogeneration und Peltier-Elemente eignen sich zur Speisung drahtloser Sensoren und insoweit zur zumindest teilweisen Eigenenergieversorgung des Systems 10 in relevanten Systemzuständen.

In Verbindung mit einer hochgenauen Hausortung, beispielsweise nach Art einer HAIP (High Accuracy Indoor Positioning), und BLE (Bluetooth Low Energy)-Konformität in Bezug auf die Eigenenergieversorgung des Systems 10, d.h. bei ausreichend geringer Stromaufnahme insbesondere des Trägermoduls 50, ist ferner mit geeigneter Antennenanordnung eine Sterilisationsbehälter-Nachverfolgung und/oder-Ortung, beispielsweise innerhalb eines Lagers, auf der Grundlage des BLE RSSI-Werts (Bluetooth Low Energy Received Signal Strength Indicator) oder einer Innenraumnavigation denkbar.

Insoweit bildet das Trägermodul 50 in diesem Ausführungsbeispiel eine Einheit, die als solche vollständig mit einem geeigneten Vergussmaterial oder Glas vergossen oder eingeschlossen ist. Das Vergussmaterial sowie alle Komponenten des Trägermoduls 50 weisen eine Temperaturbeständigkeit derart auf, dass die gesamte Einheit mit sterilisierbar ist.

Der Träger 30 ist vorzugsweise aus einem Kunststoff hergestellt und kann starr beziehungsweise steif oder auch flexibel und/oder elastisch ausgebildet sein. Eine flexible und/oder elastische Ausgestaltung ist vorzugsweise dann vorgesehen, wenn der Träger 30 in Form einer in den Sterilisationsbehälter 12 einlegbaren Matte ausgebildet ist. Das vollständig bestückte Trägermodul 50 und die Sensoren 32, soweit letztere vorhanden sind, können auf den Träger 30 aufgeklebt oder alternativ auch in nicht näher dargestellten Ausnehmungen des Trägers 30 angeordnet sein. Vorzugsweise umgibt der Träger 30 das Trägermodul 50 und die Sensoren 32 vollständig, um sie zu schützen. Vorzugsweise umgibt der Träger 30 das Trägermodul 50 und die Sensoren 32 formschlüssig. Beispielweise können das Trägermodul 50 und die Sensoren 32 zur Herstellung der Behälterinhalt-Sensoreinrichtung 28 mit dem Material, aus dem der Träger gebildet wird, umgossen oder vergossen werden.

Ist der Träger 30 in Form einer Einlegevorlage ausgebildet, sind vorzugsweise Konturen 42 oder Abbildungen der im Sterilisationsbehälter 12 zu lagernden Gegenstände 24 schematisch dargestellt. Die Konturen 42 können insbesondere auf den Träger 30 aufgedruckt, in ihn eingraviert oder durch Laserbeschriftung oder dergleichen auf ihn aufgebracht sein.

Die Sensoren 32 sind vorzugsweise innerhalb der Konturen 42 oder im Bereich derselben angeordnet, und zwar derart, dass sie in einer größtmöglichen räumlichen Nähe zu den Identifizierungselementen 34 stehen, wenn die Gegenstände 24 geordnet im Siebkorb 18 gelagert sind.

Gegebenenfalls vorhandene Sensoren 32 und insbesondere der Sensor 52, die beispielsweise eine Spule umfassen können, sind elektrisch leitend mit dem drahtlos arbeitenden Funkmodul 56 verbunden. Elektrische Verbindungsleitungen zu und von den Sensoren 32 können beispielsweise auf den Träger 30 aufgedruckt oder in diesen eingegossen sein. Aufgrund der drahtlosen Datenübermittlung, die eine Kopplung mit der vom System umfassten Auslese- und/oder Auswerteeinheit 62 außerhalb des Sterilisationsbehälters 12 herstellt und unterhält, kann der Träger 30 wahlfrei in dem Sterilisationsbehälter 12 angeordnet werden, d.h. entweder am Behälterunterteil 14, am Behälteroberteil 16, oder direkt im Siebkorb 18.

Die Auslese und/ oder Auswerteeinheit 62 kann insbesondere eine Anzeige 64 umfassen, um den Inhalt des Sterilisationsbehälters 12 grafisch oder in Form einer Liste darzustellen. Die Auslese- und/oder Auswerteeinheit 62 beinhaltet darüber hinaus Funktionen eines Computersystems, die eine Nachverfolgung der Gegenstände 24, beispielsweise in einem Krankenhaus, ermöglichen bzw. zumindest unterstützen.

Das oben beschriebene System 10 ermöglicht somit eine Kopplung der Auslese- und/oder Auswerteeinheit 62 mit vorzugsweise jedem der vorgesehenen Sensoren 32 der Behälterinhaltsensoreinrichtung 28. Dies ist dienlich, um von dem oder den Sensoren 32 erzeugte Detektionssignale zur Auslese- und/oder Auswerteeinheit 62 zu übertragen zum Ermitteln des Inhalts des Sterilisationsbehälters 12. Die Übertragung erfolgt über eine drahtlose Verbindung, beispielsweise durch eine Funkverbindung wie etwa Bluetooth, um Detektionssignale der Sensoren 32 an die Auslese- und/oder Auswerteeinheit 62 zu übermitteln. Das Trägermodul 50 umfasst in diesem Fall eine für eine berührungslose Übertragung der Detektionssignale geeignete Sende- und Empfangseinheit.

Das beschriebene System 10 umfasst somit ein eine TransponderLeseeinrichtung bildendes bzw. umfassendes Trägermodul 50 zum Erfassen eines Identifizierungselements 34 und Übertragen erfasster Daten mittels funkbasierter Datenübertragung aus dem Sterilisationsbehälter 12 heraus, und beinhaltet zum einen den mindestens einen Sensor 52 und/oder 32 und zum anderen die Auslese- und/ oder Auswerteeinheit 62. Ein oder mehrere Sensoren 32, 52 sind als Teil der Behälterinhalt-Sensoreinrichtung 28 im Inneren des Sterilisationsbehälters 12 angeordnet, um in Abhängigkeit des Vorhandenseins von Identifizierungselementen 34 von im Sterilisationsbehälter gelagerten Gegenständen 24 Erfassungssignale zu erzeugen. Die Auswertung der Erfassungssignale erfolgt dann insbesondere in der durch eine Funkstrecke durch die Gehäusewandung des Sterilisationsbehälters mit den Sensoren 32, 52 gekoppelten Auslese- und/oder Auswerteeinheit 62, welche bei einem Transponderhandlesegerät beispielsweise in einem gemeinsamen Gehäuse mit den Sensoren untergebracht wäre oder zumindest einen kabelgebundenen Anschluss erfordern würde.

Die Behälterinhalt-Sensoreinrichtung 28 im Inneren des Sterilisationsbehälters 12 anzuordnen hat insbesondere den Vorteil, dass der Behälterinhalt auch bei geschlossenem Sterilisationsbehälter 12 bestimmt werden kann, insbesondere auch dann, wenn der Sterilisationsbehälter 12 ganz aus einem Metall, beispielsweise Aluminium, hergestellt ist. Aufgrund der Abschirmwirkung von Metallen für elektromagnetische Strahlung ist eine Abfrage des Inhalts des Sterilisationsbehälters 12 mit einem handelsüblichen Handlesegerät zum Auslesen von Transpondern nicht geeignet. Die funkbasierte Ankopplung an die Auslese- und/oder Auswerteeinheit 62 ermöglicht größere Freiheitsgrade bezüglich deren konstruktiven Aufbaus und erhöht aufgrund des Entfalls eines Verbindungskabels vorbestimmter Länge den Aktionsradius der Erfassung. Ferner wird die Fehleranfälligkeit aufgrund defekter Verkabelung und defekter Steckkontakte verringert, und sind, nicht zuletzt aufgrund der Speicherung relevanter Parameter und Daten in dem Trägermodul 50 und damit unabhängig von der Einheit 62 direkt in dem Sterilisationsbehälter 12, kompatible Auslese- und Auswerteeinheiten 62 erforderlichenfalls ohne Weiteres zusätzlich und/oder parallel bzw. mehrfach ankoppelbar und/oder austauschbar.

Schließlich bildet der einlegbare Träger 30 mit darin eingebettetem Trägermodul 50 und Sensoren 32, 52 eine eigenständige, separate Einheit und Komponente des Systems 10, die aufgrund der drahtlosen Anbindung an die außenseitige Auslesung und Auswertung keinerlei weitere betriebsrelevante Verbindung zu oder Fixerung an dem Sterilisationsbehälter 12 erfordert und insoweit optional einsetzbar und/oder nachrüstbar ist.

## Patentansprüche

1. Medizinisches Behälterinhalt-Erfassungssystem, beinhaltend
eine Behälterinhalt-Sensoreinrichtung (28) zum Anordnen in einem Sterilisationsbehälter (12), welche Behälterinhaltsensoreinrichtung (28) einen Träger (30) und mindestens einen am Träger (30) angeordneten oder ausgebildeten Sensor (32, 52) zum Erfassen mindestens eines Identifizierungselements (34), welches an einem im Sterilisationsbehälter (12) gelagerten Gegenstand (24) zu dessen Identifikation angeordnet oder ausgebildet ist, umfasst; und ein an dem Träger (30) angeordnetes Trägermodul (50), das eine Erfassungseinrichtung zur Erfassung mindestens eines erfassten Identifizierungselements (34) aufweist und dazu angeordnet ist, Information über den durch das erfasste Identifizierungselement (34) identifizierten Gegenstand (24) drahtlos nach außerhalb des Sterilisationsbehälters (12) zu übermitteln;
**gekennzeichnet durch**
einen Siebkorb (18) mit mindestens einer Lagereinrichtung (20) für mindestens einen im Siebkorb (18) zu lagernden Gegenstand (24), welcher Siebkorb (18) in den Sterilisationsbehälter (12) einbringbar ist, wobei
die Behälterinhalt-Sensoreinrichtung (28) in dem Siebkorb (18) angeordnet oder gehalten ist; und
der Siebkorb (18) oder ein Abschnitt des Siebkorbs (18) eine Antenne bildet oder als Antenne verwendbar ist, in welche ein Erfassungssignal von zumindest einem erfassten Identifizierungselement (34) einkoppelbar ist.

2. Medizinisches Behälterinhalt-Erfassungssystem nach Anspruch 1, **dadurch gekennzeichnet, dass** die Behälterinhalt-Sensoreinrichtung (28) mindestens eine Datenübermittlungseinrichtung (56) und eine drahtlose Schnittstelle beinhaltet zur Übertragung und/oder Auskopplung von mit dem mindestens einen Sensor (32, 52) erzeugten Erfassungssignalen an eine behälterexterne Auslese- und/oder Auswerteeinheit (62) zum Bestimmen der im Sterilisationsbehälter (12) gelagerten Gegenstände (24) in Abhängigkeit von mit dem mindestens einen Sensor (32, 52) erzeugten Erfassungssignalen.

3. Medizinisches Behälterinhalt-Erfassungssystem nach Anspruch 1, **dadurch gekennzeichnet, dass** das Trägermodul (50) zumindest einen Sensor (52), der eine Transponder-Leseeinrichtung bildet eine Energieversorgungseinrichtung (54) zur Energieversorgung der Behälterinhalt-Sensoreinrichtung (28), und/oder eine drahtlos arbeitende Datenübermittlungseinrichtung (56) zur Übermittlung von erfassten Daten und/oder Erfassungssignalen an eine behälterexterne Auslese- und/oder Auswerteeinrichtung (62) beinhaltet, und das Trägermodul (50) zu einer dampfsterilisierbaren Einheit vergossen und/oder durch Einschließen zusammengefasst ist.

4. Medizinisches Behälterinhalt-Erfassungssystem nach Anspruch 1, **dadurch gekennzeichnet, dass** eine Temperatur-, Feuchtigkeit- und Druckhöhenerfassungseinrichtung vorgesehen ist, zur Erfassung und Aufnahme von Temperatur, Feuchtigkeit und Druck während eines Sterilisationsprozesses, wobei die Temperatur-, Feuchtigkeit- und Druckhöhenerfassungseinrichtung im/am Trägermodul (50) angeordnet ist.

5. Medizinisches Behälterinhalt-Erfassungssystem nach Anspruch 1, **dadurch gekennzeichnet, dass** das Trägermodul (50) eine Speichereinrichtung beinhaltet zur Speicherung fester Daten und zumindest Zwischenspeicherung erfasster Daten, wobei die Speichereinrichtung dazu angeordnet ist, zumindest Identifikationsinformation des Sterilisationsbehälters (12), die zumindest eine Seriennummer oder eine Kennung desselben umfassen, fest zu speichern, und/oder zumindest von einer Temperatur-, Feuchtigkeit- und/oder Druckhöhenerfassungseinrichtung erzeugte Daten in Bezug auf einen Temperatur-, Feuchtigkeit- und/oder Druckhöhenverlauf an dem Sterilisationsbehälter (12), die zumindest Information über einen letzten Sterilisationszeitraum oder eine Anzahl von Sterilisationszyklen beinhalten, von dem wenigstens einen Sensor (32, 52) bezüglich der Identifizierungselemente erfasste Daten oder Daten bezüglich einer Sterilisationshistorie und/oder einer Wartungshistorie des Sterilisationsbehälters (12) zwischenzuspeichern.

6. Medizinisches Behälterinhalt-Erfassungssystem nach Anspruch 1, **dadurch gekennzeichnet, dass** das Trägermodul (50) eine Relaisfunktion beinhaltet, die für ein Aktivieren und/oder Deaktivieren von zumindest Teilen des Behälterinhalt-Sensorsystems (28) einschließlich des Trägermoduls (50) selbst konfiguriert ist, wobei die Relaisfunktion in Abhängigkeit von an dem Trägermodul (50) erfassten Zustandsänderungen eigenauslösbar oder abhängig oder unabhängig von derartigen Zustandsänderungen fernauslösbar ist.

7. Medizinisches Behälterinhalt-Erfassungssystem nach Anspruch 3, **dadurch gekennzeichnet, dass** die Energieversorgungseinrichtung (54) für eine Eigenenergieversorgung der Behälterinhalt-Sensoreinrichtung (28) angeordnet ist und zumindest eine wiederaufladbare Energiespeichereinrichtung umfasst, die induktiv und/oder mittels Thermogeneration behälterseitig erzeugter elektrischer Energie speisbar ist.

8. Medizinisches Behälterinhalt-Erfassungssystem nach einem der vorangehenden Ansprüche, **dadurch gekennzeichnet, dass** das Trägermodul (50) dazu angeordnet ist, in Verbindung mit einer hochgenauen Hausortung und BLE-Konformität in Bezug auf die Eigenenergieversorgung der Behälterinhalt-Sensoreinrichtung (28) eine Einrichtung zur Sterilisationsbehälter-Nachverfolgung und/oder -Ortung auf der Grundlage eines RSSI-Werts und/oder eine Richtungserkennungseinrichtung bereitzustellen.

9. Medizinisches Behälterinhalt-Erfassungssystem nach einem der vorangehenden Ansprüche, **dadurch gekennzeichnet, dass** der mindestens eine Sensor (32, 52) dazu angeordnet ist, Identifizierungselemente (34) in Form von Transpondern zu erfassen.

10. Medizinisches Behälterinhalt-Erfassungssystem nach einem der vorangehenden Ansprüche, **dadurch gekennzeichnet, dass** der mindestens eine Sensor (32, 52) in Form eines Transponders ausgebildet und in einer Ausnehmung des Trägers (30) aufgenommen ist.

11. Medizinisches Behälterinhalt-Erfassungssystem nach einem der vorangehenden Ansprüche, **dadurch gekennzeichnet, dass** der Träger (30) das Trägermodul (50) und den mindestens einen Sensor (32, 52) mindestens teilweise formschlüssig umgibt und aus einem dampfsterilisierbaren Material hergestellt ist.

12. Medizinisches Behälterinhalt-Erfassungssystem nach einem der vorangehenden Ansprüche, **dadurch gekennzeichnet, dass** der Träger (30) aus einem Glas- oder Kunststoffmaterial hergestellt ist.

13. Medizinisches Behälterinhalt-Erfassungssystem nach einem der vorangehenden Ansprüche, **dadurch gekennzeichnet, dass** der Träger (30) in Form einer Matte ausgebildet ist.

14. Medizinisches Behälterinhalt-Erfassungssystem nach einem der vorangehenden Ansprüche, **dadurch gekennzeichnet, dass** der Träger (30) in Form einer Einlegevorlage ausgebildet ist, auf welcher im Sterilisationsbehälter (12) zu lagernde Gegenstände (24) mindestens schematisch dargestellt sind.

15. Medizinisches Behälterinhalt-Erfassungssystem nach einem der vorangehenden Ansprüche, **gekennzeichnet durch** einen Sterilisationsbehälter (12) mit einem Behälterunterteil (14) und einem Behälteroberteil (16) zum Verschließen des Behälterunterteils (14), wobei die Behälterinhalt-Sensoreinrichtung (28) mechanisch verbindungsfrei in den Sterilisationsbehälter (12) einlegbar ist.

## Claims

1. A medical container content detection system, comprising
a container content sensor device (28) for arrangement in a sterilization container (12), wherein the container content sensor device (28) comprises a carrier (30) and at least one sensor (32, 52) arranged or formed on the carrier (30) for detecting at least one identification element (34) which is arranged or formed on an object (24) stored in the sterilization container (12) for the identification thereof, and
a carrier module (50) arranged on the carrier (30) which includes a detection device for detecting at least one detected identification element (34) and is arranged for the purpose of wirelessly transmitting information about the object (24) identified by the detected identification element (34) to the outside of the sterilization container (12);
**characterized by**
a screen basket (18) comprising at least one storing means (20) for at least one object (24) to be stored in the screen basket (18), said screen basket (18) being adapted to be introduced to the sterilization container (12), wherein
the container content sensor device (28) is arranged or retained in the screen basket (18),
the screen basket (18) or a section of the screen basket (18) forms an antenna or can be used as antenna, into which a detection signal of at least one detected identification element (34) can be coupled.

2. The medical container content detection system according to claim 1, **characterized in that** the container content sensor device (28) includes at least one data transmission device (56) and a wireless interface for transmitting and/or extracting detection signals generated by the at least one sensor (32, 52) to a read-out and/or evaluating unit (62) external to the container for determining the objects (24) stored in the sterilization container (12) in response to detection signals generated by the at least one sensor (32, 52).

3. The medical container content detection system according to claim 1, **characterized in that** the carrier module (50) includes at least one sensor (52) forming a transponder reading device and/or an energy supply unit (54) for supplying the container content sensor device (28) with energy and/or a wirelessly operating data transmission device (56) for transmitting detected data and/or detection signals to a read-out and/or evaluating unit (62) external to the container, wherein the carrier module (50) is cast to form a steam-pressure sterilizable unit and/or is preferably combined by enclosing.

4. The medical container content detection system according to claim 1, **characterized in that** a temperature, humidity and pressure level detection device is provided for detecting and recording temperature, humidity and pressure during a sterilization process, wherein the temperature, humidity and pressure level detection device is arranged in/on the carrier module (50).

5. The medical container content detection system according to claim 1, **characterized in that** the carrier module (50) includes a storage unit for storing fixed data and at least intermediately storing detected data, wherein the storage unit is arranged for permanently storing at least identification information of the sterilization container (12) including at least a serial number or an identification of the same, and/or for intermediately storing at least data generated by a temperature, humidity and/or pressure level detection device in relation to a temperature, humidity and/or pressure level curve at the sterilization container (12), said data containing at least information about a recent sterilization period or a number of sterilization cycles, data detected by the at least one sensor (32 52) relating to the identification elements or data relating to a sterilization history and/or a maintenance history of the sterilization container (12).

6. The medical container content detection system according to claim 1, **characterized in that** the carrier module (50) includes a relay function which is configured for activating and/or deactivating at least parts of the container content sensor system (28) including the carrier module (50) itself, wherein the relay function can be self-triggered in response to changes of condition detected on the carrier module (50) or can be remote-triggered dependent on or independent of said changes of conditions.

7. The medical container content detection system according to claim 3, **characterized in that** the energy supply unit (54) is arranged for energy self-supply of the container content sensor device (28) and comprises at least one rechargeable energy storing unit which can be supplied with electrical energy generated inductively and/or by means of thermal generation on the container side.

8. The medical container content detection system according to any one of the preceding claims, **characterized in that** the carrier module (50) is arranged for providing a device for sterilization container tracking and/or positioning based on a RSSI value and/or a direction identification device in connection with a high-accuracy indoor positioning and BLE conformity in relation to the energy self-supply of the container content sensor device (28).

9. The medical container content detection system according to any one of the preceding claims, **characterized in that** the at least one sensor (32, 52) is arranged for detecting identification elements (34) in the form of transponders.

10. The medical container content detection system according to any one of the preceding claims, **characterized in that** the at least one sensor (32, 52) is in the form of a transponder and is received in a recess of the carrier (30).

11. The medical container content detection system according to any one of the preceding claims, **characterized in that** the carrier (30) surrounds the carrier module (50) and the at least one sensor (32, 52) at least partially by form closure and is made from steam-pressure sterilizable material.

12. The surgical container content detection system according to any one of the preceding claims, **characterized in that** the carrier (30) is made from glass or plastic material.

13. The medical container content detection system according to any one of the preceding claims, **characterized in that** the carrier (30) is in the form of a mat.

14. The medical container content detection system according to any one of the preceding claims, **characterized in that** the carrier (30) is in the form of an insertion pattern on which objects (24) to be stored in the sterilization container (12) are shown at least schematically.

15. The medical container content detection system according to any one of the preceding claims, **characterized by** a sterilization container (12) comprising a lower container part (14) and an upper container part (16) for closing the lower container part (14), wherein the container content sensor device (28) can be inserted in the sterilization container (12) free from mechanical connections.

## Revendications

1. Système de détection de contenu de conteneur médical comprenant un moyen capteur de contenu de conteneur (28) destiné à être placé dans un conteneur de stérilisation (12), lequel moyen capteur de contenu de conteneur (28) comprend un support (30) et au moins un capteur (32, 52) agencé ou formé sur le support (30) pour détecter au moins un élément d'identification (34) qui est agencé ou formé sur un article (24) monté dans le conteneur de stérilisation (12) pour son identification ; et
un module de support (50) disposé sur le support (30), doté d'un dispositif de détection pour détecter au moins un élément d'identification détecté (34) et conçu pour transmettre des informations sur l'article (24) identifié par l'élément d'identification détecté (34) sans fil à l'extérieur du conteneur de stérilisation (12) ;
**caractérisé par** un panier à tamis (18) avec au moins un dispositif de stockage (20) pour au moins un article (24) à stocker dans le panier à tamis (18), lequel panier à tamis (18) peut être introduit dans le conteneur de stérilisation (12), dans lequel le moyen capteur de contenu du conteneur (28)) est disposé ou maintenu dans le panier à tamis (18) ; et
le panier à tamis (18) ou une partie du panier à tamis (18) forme une antenne ou peut être utilisé(e) en tant qu'antenne à laquelle un signal de détection d'au moins un élément d'identification détecté (34) peut être couplé.

2. Système de détection de contenu de conteneur médical selon la revendication 1, **caractérisé en ce que** le moyen capteur de contenu de conteneur (28) comprend au moins un dispositif de transmission de données (56) et une interface sans fil pour la transmission et/ou le découplage de signaux de transmission générés avec le au moins un capteur (32, 52) à/d'une unité externe de lecture et/ou d'évaluation (62) pour déterminer les articles (24) stockés dans le conteneur de stérilisation (12) en fonction des signaux de détection générés par le au moins un capteur (32, 52).

3. Système de détection de contenu de conteneur médical selon la revendication 1, **caractérisé en ce que** le module de support (50) comporte au moins un capteur (52), qui forme un dispositif de lecture-transpondeur, un dispositif d'alimentation en énergie (54) pour fournir de l'énergie au moyen capteur de contenu de conteneur (28), et/ou un dispositif de transmission de données sans fil (56) pour transmettre des données acquises et/ou des signaux de détection à un dispositif de lecture et/ou d'évaluation externe (62) et pour intégrer et/ou regrouper par enfermement le module de support (50) dans une unité stérilisable à la vapeur.

4. Système de détection de contenu de conteneur médical selon la revendication 1, **caractérisé en ce qu'**un dispositif de détection de température, d'humidité et de hauteur de pression est prévu pour détecter et enregistrer la température, l'humidité et la pression d'un processus de stérilisation, dans lequel le dispositif de détection de température, d'humidité et de pression de hauteur dans/est disposé sur le module porteur (50).

5. Système de détection de contenu de conteneur médical selon la revendication 1, **caractérisé en ce que** le module de support (50) comprend un dispositif de stockage destiné à stocker des données permanentes et à au moins une mise en mémoire tampon de données détectées, dans lequel le dispositif de stockage est agencé pour stocker de manière permanente au moins une information d'identification du conteneur de stérilisation (12), qui comprend au moins un numéro de série ou son identifiant, et/ou pour mettre en mémoire tampon au moins des données générées par un dispositif de détection de température, d'humidité et/ou d'altitude-pression en ce qui concerne une courbe de température, d'humidité et/ou d'altitude-pression sur le conteneur de stérilisation (12), la au moins une information concernant une dernière période de stérilisation ou un nombre de cycles de stérilisation, provenant du au moins un capteur (32, 52) en ce qui concerne les éléments d'identification, des données détectées ou des données relatives à un historique de stérilisation et/ou à un historique de maintenance du conteneur de stérilisation (12).

6. Système de détection de contenu de conteneur médical selon la revendication 1, **caractérisé en ce que** le module de support (50) comprend une fonction de relais configurée pour activer et/ou désactiver au moins des parties du système de capteur de contenu de conteneur (28) comprenant le module de support (50) lui-même, dans lequel la fonction de relais est déclenchée en fonction de changements d'état détectés sur le module de support (50), ou de manière dépendante ou indépendante par rapport à de tels changements d'état,

7. Système de détection de contenu de conteneur médical selon la revendication 3, **caractérisé en ce que** le dispositif d'alimentation en énergie (54) est conçu pour alimenter en énergie le moyen capteur de contenu de conteneur (28), et comprend au moins un dispositif de stockage d'énergie rechargeable qui peut être alimenté en énergie électrique générée côté conteneur par induction et/ou thermogénération.

8. Système de détection de contenu de conteneur médical selon l'une des revendications précédentes, **caractérisé en ce que** le module de support (50) est agencé, en liaison avec une localisation extrêmement précise et une conformité BLE par rapport à l'alimentation en énergie propre du moyen capteur de contenu de conteneur (28), pour fournir un suivi et/ou une localisation du conteneur de stérilisation sur la base d'une valeur RSSI et/ou d'un dispositif de détection de direction.

9. Système de détection de contenu de conteneur médical selon l'une quelconque des revendications précédentes, **caractérisé en ce que** le au moins un capteur (32, 52) est conçu pour détecter des éléments d'identification (34) sous la forme de transpondeurs.

10. Système de détection de contenu de conteneur médical selon l'une des revendications précédentes, **caractérisé en ce que** le au moins un capteur (32, 52) est réalisé sous la forme d'un transpondeur et inclus dans un renfoncement du support (30).

11. Système de détection de contenu de conteneur médical selon l'une des revendications précédentes, **caractérisé en ce que** le support (30) entoure au moins partiellement le module de support (50) et le au moins un capteur (32, 52) de manière ajustée, et est fabriqué à partir d'un matériau pouvant être stérilisé à la vapeur.

12. Système de détection de contenu de conteneur médical selon l'une quelconque des revendications précédentes, **caractérisé en ce que** le support (30) est fabriqué en verre ou en matière plastique.

13. Système de détection de contenu de conteneur médical selon l'une des revendications précédentes, **caractérisé en ce que** le support (30) se présente sous la forme d'un mat.

14. Système de détection de contenu de conteneur médical selon l'une des revendications précédentes, **caractérisé en ce que** le support (30) est conçu sous la forme d'un modèle pouvant être inséré sur lequel des articles (24) à stocker dans le conteneur de stérilisation (12) sont représentés au moins schématiquement.

15. Système de détection de contenu de conteneur médical selon l'une des revendications précédentes, **caractérisé par** un conteneur de stérilisation (12) avec une partie inférieure de conteneur (14) et une partie supérieure de conteneur (16) pour fermer la partie inférieure de conteneur (14), dans lequel le moyen capteur de contenu de conteneur (28) peut être agencé sans connexion mécanique dans le conteneur de stérilisation (12).
